# EUROPÄISCHE PATENTANMELDUNG

(11) **EP 2 157 181 A1**
(43) Veröffentlichungstag der Anmeldung: **24.02.2010**
(21) Anmeldenummer: 08162327.4
(22) Anmeldetag: 13.08.2008
(51) Int. Cl.: C12N 15/10, C12N 15/09

(54) **Verfahren zur Isolation von Nukleinsäuren und Testkit**

(71) Anmelder: AGOWA Gesellschaft für molekularbiologische Technologie mbH, 12459 Berlin (DE)
(72) Erfinder: Schubert, Frank, Dr., 12623 Berlin (DE); Fartmann, Bertold, 12524 Berlin (DE)
(74) Vertreter: Ziebig, Marlene

(57) **Zusammenfassung**

Die Erfindung betrifft ein Verfahren zur Isolation von Nukleinsäuren, wobei zur Bindung der Nukleinsäuren ein Trägermaterial verwendet wird, das eine Oberfläche aufweist, die mindestens zwei chemisch unterscheidbare, nicht zu einer Stoffklasse gehörende funktionelle Gruppen "X" und "Y" besitzt, die beide eine sehr schwache bis keine (inherente) Affinität zu Nukleinsäuren besitzen sowie einen Testkit zur Durchführung des Verfahrens.

## Beschreibung

Die Erfindung betrifft ein Verfahren zur Isolation von Nukleinsäuren, wobei zur Bindung der Nukleinsäuren ein Trägermaterial verwendet wird, das eine Oberfläche aufweist, die mindestens zwei chemisch unterscheidbare, nicht zu einer Stoffklasse gehörende funktionelle Gruppen "X" und "Y" besitzt, die beide eine sehr schwache bis keine (inherente) Affinität zu Nukleinsäuren besitzen sowie einen Testkit zur Durchführung des Verfahrens.

Mit der Etablierung nukleinsäureanalytischer Methoden in der klinischen und molekularbiologischen Laboratoriumspraxis, insbesondere der klinischen Analytik, haben Methoden zur Nukleinsäureaufreinigung eine rasante technologische Entwicklung durchlaufen. Besonderes Augenmerk galt vor allem Nukleinsäureisolationsverfahren, die geeignet sind auf automatischen "Liquid Handling"-Systemen appliziert zu werden. Herausragend sind hier Festphasenextraktionskonzepte, d.h. die aus dem zu untersuchenden Probenmaterial zu isolierenden Nukleinsäuren werden an feste Oberflächen gebunden, in der Probenmatrix enthaltene Verunreinigungen aus dem heterogenen System heraus gewaschen und anschließend die gereinigten Nukleinsäuren von der festen Phase wieder abgelöst.

Bislang bekannte Verfahren nutzten im wesentlichen die Oberflächeneigenschaften von weitestgehend homogenen chemischen Stoffen, wie beispielsweise Glasfasern oder silikatische Materialien oder native bzw. synthetische homogene Adsorbentien wie Apatit.

Eine außerordentliche Stellung haben Adsorbenzien in Form von superparamagnetischen Mikropartikeln, da durch die mögliche Manipulation dieser Adsorbenzien mit externen magnetischen Feldern ein manuelles Eingreifen in den Extraktionsverlauf umgangen und der Prozeß damit besonders vorteilhaft vollautomatisch gestaltet werden kann.

Eines der ältesten Verfahren zur Nukleinsäureisolation ist die von Gillespie und Vogelstein vorgeschlagene Anwendung von silikatischen Materialien, wie fein verteiltem Glas, an welche Nukleinsäuren in Pufferlösungen fixiert werden, die im wesentlichen chaotrope Salze als Pufferbestandteil verwenden. Van Boom hat dieses Verfahren unter Nutzung ähnlicher silicatischer Matrices und Puffersysteme auf komplexere biologische Materialien erweitert [EP 389 063 A2]. Nachteilig in diesen Systemen ist vor allem, dass die Nukleinsäuren nur unter den chaotrophen Bedingungen, die zu ihrer Bindung an die festen Oberflächen geführt haben, auch an den Oberflächen verbleiben. Die zur Bindung der Nukleinsäuren notwendigen chaotropen Salze stören aber empfindlich die an die Nukleinsäuerisolierung folgende eigentliche Analytik. Deshalb wurden diese störenden chaotropen Salze bislang durch den Einsatz von Waschlösungen die einen erheblichen Anteil von in Wasser löslichen organischen Lösungsmitteln haben (meist deutlich mehr als 50 vol% organisches Lösungsmittel) von der festen Phase gewaschen, wobei die zu isolierenden Nukleinsäuren an der festen Phase gebunden bleiben, da sie in den verwendeten Waschpuffern nicht löslich sind.
Diese organischen Lösungsmittel haben jedoch auch ein ebenfalls erhebliches Inhibitionspotential für die vor allem auf enzymatischen Reaktionen beruhende Nukleinsäureanalytik und müssen deshalb vor der eigentlichen Elution der Nukleinsäuren entfernt werden, was den Prozess erheblich verlangsamt und technisch kompliziert (Temperaturerhöhung um Verdampfen zu beschleunigen, lange Wartezeiten).
Auch ist das Verdampfen organischer Lösungsmittel in geschlossenen Systemen, etwa einer Mikroreaktionskammer oder einem Reagentienchip zum Amplifizieren und zur Analyse von Nukleinsäuren sehr schwierig, wenn nicht unmöglich.

Ein auf der Nutzung nichtchaotroper Salze beruhendes Verfahren begründen Bendzko et al. [DE 19856064 A1], in dem sie sogenannte kosmotrope Salzlösungen zur Bindung von Nukleinsäuren an silikatische Oberflächen nutzen, ohne dabei jedoch die prinzipiellen Nachteile des Ursprungverfahrens, wie den notwendigen Einsatz organischer Lösungsmittel in den Waschprozessen, zu beseitigen. Entsprechend dieser Nachteile wird seit langem nach alternativen Bindungsmöglichkeiten von Nukleinsäuren an feste Oberflächen gesucht.

Die Nutzung von negativ geladenen, Karboxylgruppen tragenden magnetischen Mikropartikeln zur Isolierung von DNA ist durch Hawkins ausführlich beschrieben [WO 9609379 A] worden. Dabei wird die DNA unter Verwendung von Puffersystemen mit hohem Anteil von Polyethylenglycol und nicht chaotropen Salzen an karboxylierte Oberflächen gebunden. Auch hier ist der Einsatz von organischen Lösungsmitteln in den Waschpuffern erforderlich, um die Komponenten der Bindungspuffer aus dem System zu entfernen. Ein ähnlicher Ansatz wird in WO 02066993 A verfolgt. Anstelle der Karboxylgruppen tragenden magnetischen Mikropartikel werden hier Zellulosederivate mit speziellen magnetischen Eigenschaften zur Nukleinsäureisolation mit Hilfe von polyethylenglycolhaltigen Puffern vorschlagen.

Die Verwendung von silikatischen Materialien in Kombination mit Puffersystemen, die organische Lösungsmittel enthalten, wird in EP 0512 767 A2 offenbart. Hier spielen die Polaritätsverhältnisse der Komponenten im verwendeten heterogenen System eine entscheidende Rolle. Ebenfalls müssen die organischen Lösungsmittel durch Evaporation vor der eigentlichen Elution der Nukleinsäuren aus dem System entfernt werden, da sonst die sich der Isolation anschließende Nukleinsäureanalytik gefährdet ist.

Methodisch grundsätzlich anders ist das Verfahren, welches den so genannten Charge Switch Mechanismus verwendet [WO 0248164 A]. Hier werden Nukleinsäuren in einem wässrigen Puffersystem mit einem schwach sauren pH-Wert unter Nutzung eines Ionenaustauschmechanismus an positiv geladene Oberflächen gebunden. Bei Beibehaltung des schwach sauren pH-Wertes, werden in rein wässrigem Medium unter kontrollierter Ionenstärke die Verunreinigungen aus dem System heraus gewaschen und anschließend durch Einsatz schwach alkalischer Bedingungen die Nukleinsäuren vom Träger eluiert. Dies gelingt aufgrund der in das System eingebrachten ionenaustauschaktiven Gruppen (i.d.R. aliphatische oder heterocyclische Aminogruppen), deren Ladung in Abhängigkeit vom pH-Wert des Mediums positiv oder neutral ist. Die Bindung der Nukleinsäuren an die Oberflächen erfolgt dabei direkt unter Nutzung von ionischen Wechselwirkung der negativ geladenen Nukleinsäuren mit der positiv geladenen Oberfläche der beschriebenen Partikel/Oberflächen.
Problematisch ist in diesem System, dass erstens eine Anbindung aller in der Probenmatix enthaltenen Makromoleküle mit einer negativen Nettoladung wahrscheinlich ist und damit die Koisolation von Proteinen und negativ geladenen Polysacchariden, wie z.B. Heparin, droht, was die Qualität der isolierten DNA negativ beeinflusst. Auch sind Ionenaustauschwechselwirkungen sehr empfindlich gegenüber höheren Ionenstärken und der Anwesenheit von Detergenzien im Bindungsmedium. Beides erfordert ein sehr genaues Einstellen der Bindungsbedingungen (neben dem pH-Wert besonders die Salzkonzentration und den Gehalt von z.B. kationischer Detergenzien), was Kompromisse bei der Ausgestaltung der Lysebedingungen zum Aufschluss des Probenmaterials erzwingt. Demgegenüber sind die eingangs beschriebenen, auf polaren Wechselwirkungen beruhenden Mechanismen wesentlich robuster, was erhebliche Konsequenz für die eingesetzten Lysepuffer- und Bindungspuffersysteme hat.

In jüngerer Zeit wird gezielt nach Oberflächenstrukturen gesucht, die die Isolation von Nukleinsäuren unter Bedingungen erlauben, die unabhängig vom verwendeten Basismaterial sind.

Aufgabe der vorliegenden Erfindung ist es deshalb, ein Verfahren zur Isolation von Nukleinsäuren bereitzustellen, das eine komplexe Nukleinsäureanalytik ermöglicht. Dabei sollen Trägermaterialien verwendet werden, deren chemische Modifikation Oberflächeneigenschaften gewährleisten, die eine große materialtechnische Flexibilität gestatten und so bei der Entwicklung moderner, integrierter Systeme zur Nukleinsäureanalytik, wie zum Beispiel Biochips, Microfluidsysteme und ähnlichen Systemen zur Anwendung kommen können.

Die Aufgabe wird erfindungsgemäß durch ein Verfahren gelöst, welches dadurch gekennzeichnet ist, dass zur Bindung der Nukleinsäuren ein Trägermaterial verwendet wird, das eine Oberfläche aufweist, die mindestens zwei chemisch unterscheidbare, nicht zu einer Stoffklasse gehörende funktionelle Gruppen "X" und "Y" besitzt, die beide in einer bekannten chemischen Umgebung eine sehr schwache bis keine (inherente) Affinität zu Nukleinsäuren besitzen. Das Verfahren ist **dadurch gekennzeichnet, dass** ein die Nukleinsäuren enthaltendes Probenmaterial mit dem modifizierten Trägermaterial inkubiert wird. Die Bedingungen werden so eingestellt, dass die beiden funktionellen Gruppen unterschiedliche Affinitäten zu den Nukleinsäuren entwickeln und die Nukleinsäuren nacheinander an die funktionelle Gruppe "X" und "Y" gebunden werden. Die erfindungsgemäß eingesetzten Adsorptionsmaterialien besitzen auf ihrer Oberfläche die unterschiedlichen funktionellen Gruppen "X" und "Y", die chemisch nicht miteinander verwandt sind und in verschiedenen chemischen Umgebungen unterschiedlich starke Affinitäten zu den Nukleinsäuren haben.

Das Trägermaterial weist mindestens zwei funktionelle Gruppen auf der Oberfläche auf. Eine Gruppierung, die funktionelle Gruppe "X", ist vorzugsweise zur Ausbildung polarer Wechselwirkungen geeignet. Dabei kann es sich um Gruppen handeln, die zur Ausbildung nichtionischer Wechselwirkungen befähigt sind, insbesondere zur Ausbildung von Wasserstoffbrückenbindungen und van der Waals Wechselwirkungen. Vorzugsweise stellt die Gruppe "X" gebundene Hydroxyl- oder Mercaptogruppen oder Derivate dieser Gruppen dar.

Die zweite Gruppierung, die funktionelle Gruppe "Y", ist von der funktionellen Gruppe "X" chemisch klar unterscheidbar und ist hauptsächlich zur Ausbildung ionischer Wechselwirkungen befähigt. Vorzugsweise handelt es sich bei der funktionellen Gruppe "Y" um schwache ionenaustauschaktive organische Gruppen, wie z.B. Karbonsäuregruppen, Borsäure- oder Phosphorsäuregruppen.

In einem ersten Schritt wird die Nukleinsäure aus der Probenlösung an die Gruppe "X" gebunden. Eine Änderung der chemischen Umgebung des verwendeten Trägermaterials erlaubt dann eine selektive Absorption der Nukleinsäuren an die funktionelle Gruppe "Y". Unter Nukleinsäuren werden Desoxyribonuklein-, Ribonuklein- und Peptidnukleinsäuren verstanden (DNA, RNA, PNA) mit einer bevorzugten Kettenlänge von mehr als 20 Basenpaaren.

Im initialen Bindungsschritt des erfindungsgemäßen Verfahrens erfolgt die Absorption der Nukleinsäuren an die vorzugsweise polaren, funktionellen Gruppen "X" auf der Oberfläche der verwendeten festen Phase, ggf. nach einem chemischen, enzymatischen und/oder mechanischen Aufschluss. Dabei werden die Bedingungen vorzugsweise so gewählt, dass die Polarität der das Absorptionsmaterial umgebenden flüssigen Phase, der die Nukleinsäure enthaltenden Probe, niedriger ist als die des festen Trägermaterials. Die Einstellung der Polarität zur Adsorption der Nukleinsäure an die Gruppe "X" erfolgt bevorzugt unter Einwirkung von anorganischen oder organischen Salzen in Kombination mit Detergenzien und/oder durch Zugabe von mit Wasser mischbaren organischen Lösungsmitteln. Die Bedingungen kann man insbesondere mit Wasser mischbaren Alkoholen und/oder durch die Zugabe von ionischen oder nichtionischen Tensiden zum nukleinsäurehaltigen Bindungsgemisch einstellen.

Nach dem initialen Bindungsschritt erfolgt in einem der folgenden Waschschritte der erfindungsgemäß verwendeten Oberfläche eine grundsätzliche Änderung der chemischen Zusammensetzung des (das Trägermaterial mit der an "X" haftenden Nukleinsäure umgebenen) Mediums, wodurch die Spezifität der gebundenen Nukleinsäuren zur funktionellen Gruppe "X" aufgehoben und ohne die Nukleinsäuren von der Oberfläche wieder abzulösen eine Spezifität zur funktionellen Gruppe "Y" aufgebaut wird. Das kann in einem besonderen Ausführungsfall des erfindungsgemäßen Verfahrens durch die Zugabe von Substanzen erreicht werden, die eine Affinität zur funktionellen Gruppe "Y" auf der erfindungsgemäßen Oberfläche und zur Nukleinsäure haben und so die Nukleinsäure mit der funktionellen Gruppe vernetzen können.

Funktionelle Gruppen "X" in Sinne der Erfindung sind vorzugsweise an polymere organische oder anorganische Netzwerke gebundene Hydroxyl- oder Mercaptogruppen, bzw. Derivate dieser funktionellen Gruppen wie aliphatische oder aromatische Ether bzw. Thioethergruppierungen. Die funktionellen Gruppen "X" der im erfindungsgemäßen Verfahren verwendeten Adsorbentien können unmittelbar zur nativen chemischen Struktur des Adsorbens gehören, vorzugsweise handelt es sich z.B. um die Hydroxylgruppen der Zellulose und um modifizierte vernetzte Dextrane, um die Hydroxylgruppen gelartiger anorganischer Substanzen wie z.B. basische Oxidniederschläge der Form Meₘ[(O)ₙ(OH)ₚ] wobei Me ein Metallion ausgewählt aus der Reihe zwei und dreiwertiger Ionen der Übergangsmetalle, insbesonder Metalle der sechsten bis achten Nebengruppe, ganz besonders Eisen und Eisenmetalle, sein kann.
Die funktionellen Gruppen "X" können aber auch im Zuge der chemischen Modifikation indifferenter Trägermaterialien in Form geeigneter monomerer oder polymerer Strukturen auf diese indifferenten Träger aufgetragen werden.

Funktionelle Gruppen "Y" sind vorzugsweise organische Gruppierungen mit schwachen Ionenaustauschereigenschaften, vorzugsweise zur Ausbildung von Anionen befähigte Gruppen, wie z.B. Karboxylgruppen, Borsäurereste, Phosphorsäure- oder Phosphonsäurereste.

Vorzugsweise werden Trägermaterialien im erfindungsgemäßen Verfahren verwendet, die über Hydroxyl- und Karboxylgruppen verfügen.

Zur Nukleinsäureisolation nach dem erfindungsgemäßen Verfahren wird die nukleinsäurehaltige Lösung, vorzugsweise eine durch eine enzymatische Behandlung in einem Lysepuffer oder durch einen mechanischen Aufschluss vorbehandelte biologische Probenlösung, mit einem Bindungspuffer in Gegenwart der festen Trägermaterialien (Adsorbentien) mit den auf ihrer Oberfläche oder in unmittelbarer Nähe zur Oberfläche befindlichen chemisch unterscheidbaren funktionellen Gruppen "X" und "Y" gemischt.

Eine biologische Probe können Körperflüssigkeiten wie z.B. Blut oder dessen Bestandteile, Urin, Teile einer Pflanze, Materialien aus der forensischen und klinischen Analytik, insbesondere Schleimhautabstriche von Wangenschleimhäuten, getrocknete Blutspuren, Haare und Epitelzellen sein.

Puffersysteme zum Lysieren nukleinsäurehaltigen Materials sind dem Fachmann bekannt, sie enthalten bevorzugt organische und anorganische Salze und Detergenzien. Geeignete Puffersysteme sind unter anderem in molekularbiologischen Standardwerken wie "Molecular Cloning:a laboratory manual" (3rd. Edition, Sambrook, David W. Russell, ISBN 0-87969-576-5) beschrieben.

Bevorzugte in Lysepuffern zum Einsatz kommende nicht ionische Detergenzien sind die aus der Reihe der mit dem Trivialnamen Tween vertriebenen, besonders bevorzugt Tween 20. Als ionische Detergenzien besonders geeignet sind Derivate von Karbonsäuren, insbesondere das Natriumsalz des N-Laurylsarcosins.

Das nach Zugabe von Bindungspuffer zur lysierten Probe entstehende Gemisch kann neben den Bestandteilen des Puffers, der zum Lysieren der biologischen Probe eingesetzt wurde, mindestens ein chaotropes Salz in einer Konzentration von 1 - 2 Mol/l, vorzugsweise 1,5 Mol/l, ein mit Wasser mischbares organisches Lösungsmittel in einer Konzentration von 10-50vol% vorzugsweise 25-30vol%, und ein ionisches und/oder nichtionisches Detergenz mit einer Konzentration von 1-20%, vorzugsweise 3-10% enthalten. Als chaotropes Salz werden bevorzugt Guanidiniumsalze, insbesondere Guanidiniumhydrochlorid zur Anwendung gebracht.

Die funktionellen Gruppen "X" und "Y" auf dem im erfindungsgemäßen Verfahren genutzten Trägermaterial haben vorzugsweise in Lösungen, die eine Salzkonzentration von kleiner 100mM organischer oder anorganischer Salze, vorzugsweise von 0,1 bis 10mM und einen pH-Wert von größer 7,5, vorzugsweise zwischen 8 und 9 haben, keine oder nur eine sehr schwache (inherente) Affinität zu den Nukleinsäuren und können mit diesen Lösungen eluiert werden.

Für das erfindungsgemäße Verfahren ist die stoffliche Natur der festen Phase nicht erheblich sondern die Eigenschaften der Oberfläche der festen Phase.

Als feste Trägermaterialien (Adsorbentien) im Sinne des erfindungsgemäßen Verfahrens werden bevorzugt alle oberflächenmodifizierte organischen Polymere oder mineralische Materialien eingesetzt. Dem Fachmann sind Verfahren zur Aufbringung funktioneller Gruppen auf Trägermaterialien hinlänglich bekannt. Beispielhaft seien hier angeführt die Aminofunktionalisierung von silikatischen Materialien oder Gläsern durch Reaktion mit 3-Aminopropyl-triethoxysilan und dem anschließenden Umsatz mit Bernsteinsäureanhydrid zur Fixierung von Karboxylgruppen oder der Reaktion von Hydroxylgruppen einer Oberfläche mit in Phosphorsäure gelösten Phosphor(V)oxid zur Aufbringung von Phosphorsäureestergruppen. Weitere Verfahren zur Oberflächenmodifikation sind in bei Weetall [Methods in Enzymology (1976) 134] ausführlich beschrieben.

Grundmaterialien für die im erfindungsgemäßen Verfahren verwendeten Trägermaterialien können organische Polymere sein, insbesondere organische Polymere mit modifizierbaren funktionellen Gruppen, beispielsweise Aminogruppen oder Hydroxylgruppen auf deren Oberfläche.

Auch anorganische mineralische Oberflächen von z.B. Kieselgelen, Kieselgelen mit superparamagnetischen Eigenschaften, als Glasplatten ausgebildete Mikroreaktoren und Glaskapillaren sind als modifizierbare Trägeroberflächen zur Nukleinsäureisolation einsetzbar. Die ursprünglich in den genannten Materialien vorhandenen Oberflächenstrukturen werden dabei durch die Oberflächenmodifikation in der Weise verändert, dass das modifizierte Material kein dem Ausgangsmaterial entsprechende Adsorbtionseigenschaften mehr besitzt, demzufolge nicht mehr als Adsorptionsmaterial mit den für die Stoffklasse typischen Adsorptionseigenschaften eingesetzt werden kann.

In einer besonderen Ausführungsform des erfindungsgemäßen Verfahrens werden Trägermaterialien mit inhärenten magnetischen Eigenschaften, besonders superparamagnetische Mikropartikel mit einem durchschnittlichen Teilchendurchmesser von 2 bis 100µm, vorzugsweise 2 bis 50µm, besonders bevorzugt von 3 bis 8µm eingesetzt. Die prinzipielle Anwendung derartiger Materialien ist beispielhaft in "Scientific and Clinical Applications of Magnetic Carriers" (Häfeli, Schütt et al., Plenum Press; New York and London 1997) dargestellt.

Der pH-Wert der Bindungsmischung beträgt zwischen 4 und 8, bevorzugt Werte zwischen 6 und 7. Die Bindung an die Gruppe "X" erfolgt vorteilhaft bei pH-Werten um den Neutralpunkt, in Abhängigkeit vom gewählten Puffersystem bzw. des Lysepuffers.
Nach Binden der Nukleinsäuren an die Gruppe "X" der modifizierten Oberfläche wird in der Regel der Überstand abgenommen. Die Bindung der zu isolierenden Nukleinsäuren an die Gruppe "Y" erfolgt durch Änderung der chemischen Umgebung, wobei die Spezifität der gebundenen Nukleinsäure zur funktionellen Gruppe "X" des Trägermaterials in der Regel aufgehoben und eine Spezifität zur Gruppe "Y" aufgebaut wird, vorzugsweise unter Ausbildung ionischer Wechselwirkungen. Die an die Oberfläche gebundenen Nukleinsäuren werden im weiteren Isolationsprozess mit einem Waschpuffer gewaschen, der erfindungsgemäß bevorzugt eine chemische Verbindung enthält, die ein bifunktionelles Reagenz darstellt und sowohl eine Affinität zu der Oberfläche des verwendeten Adsorbens und zur Nukleinsäuren besitzt. In einer bevorzugten Ausgestaltung des Verfahrens wird ein oligomeres Polyamin verwendet, besonders bevorzugt sind Derivate des Polyethylenimins, die 2 bis 6 Aminogruppen aufweisen. Als oligomeres Polyamin können Verbindungen vom Typ kurzkettiger Polyethylenimine, bevorzugt oligomere Polyamine des Types Spermin, Spermidin oder Putrescin und deren Derivate, vorzugsweise in Form ihrer Salze eingesetzt werden. Ganz besonders bevorzugt sind die Hydrochloride. Insbesondere bevorzugt sind oligomere Ethylenamine mit 4 Aminogruppen in Form ihrer Hydrochloride. Das bifunktionelle Reagenz wird in einer Konzentration von vorzugsweise 0,1 bis 20 mM verwendet. Besonders geeignet sind Konzentrationen von 0,5 -3 mM.

Der pH-Wert des Waschpuffers liegt vorzugsweise zwischen 4 und 7, bevorzugt bei Werten zwischen 6 und 7, ganz besonders bei 6,5.

Letzte Spuren von Verunreinigungen können in weiteren Waschschritten mit reinem Wasser oder salzarmen, schwach gepufferten wässrigen Lösungen entfernt werden.

Die Elution der Nukleinsäuren kann, falls gewünscht, unter Bedingungen, die die Wechselwirkung zwischen der Nukleinsäure, den funktionellen Gruppen "X" und "Y" und gegebenenfalls dem verwendeten biaffinen Reagenz (Crosslinker) aufheben, erfolgen.
Die Bedingungen werden vorzugsweise bei einem pH-Wert zwischen 8 und 10, insbesondere bei 8,5 gewählt, in einem Temperaturbereich von 20 bis 75°C, vorzugsweise im Bereich 30°C - 55°C. Beispielhaft kann der in der Molekularbiologie bekannte sogenannte Standard-TE Puffer (10mM TrisHCl, 1mM EDTA, pH 8) verwendet werden.

Die Möglichkeit der Bindung von Nukleinsäuren an Adsorbentien, welche mit organischen Säureresten modifizierte Oberflächen besitzen unter den beschriebenen Pufferbedingungen überraschte besonders, da die im erfindungsgemäßen Verfahren interagierenden Komponenten bei pH-Werten in der Nähe des Neutralpunktes teilweise negativ geladen sind (Nukleinsäuren und funktionelle Gruppe "Y") und sich elektrostatisch abstoßen, was einer Adsorption entgegenwirkt. Trotz dieses zu erwartenden Phänomens lassen sich die Nukleinsäuren überraschend nacheinander an die funktionellen Gruppen "X" und "Y" binden.

Erst das Aufheben der Wechselwirkung zwischen der funktionellen Gruppe "Y" oder des zur Vernetzung der funktionellen Gruppe "Y" mit der gebundenen Nukleinsäure verwendeten Crosslinkers, was vorteilhaft durch Erhöhung des pH Wertes, der Temperatur oder beider Parameter erfolgen kann, führt zur Elution der Nukleinsäure von der festen Oberfläche.

In einer besonderen Ausführungsform der Erfindung kann die Analytik, die sich an die Nukleinsäureisolation anschließt, auch ohne die Elution der Nukleinsäuren von der Oberfläche erfolgen. Beispielsweise ist zu einer Amplifikation der aufgereinigten Nukleinsäuren mit Hilfe der Polymerasekettenreaktion keine Elution erforderlich.
Das alle zur Amplifikation notwendigen Reagentien enthaltene Reaktionsgemisch kann direkt auf die Trägermaterialien mit der modifizierten Oberfläche und den gebundenen Nukleinsäuren gegeben werden.

Die Spezifitätsänderung im erfindungsgemäßen Nukleinsäureisolationsprozess ermöglicht ein Verfahren, das gestattet die Nukleinsäuren mit rein wässrigen Puffern, ohne Zusatz bindungsvermittelnder Substanzen zu den Waschpuffern, wie die eingangs beschriebenen chaotropen oder kosmotropen Salze oder organische Lösungsmittel, gewaschen werden können. Dabei werden alle wasserlöslichen Verunreinigungen der Probe, die nach Verfahren des Standes der Technik an der festen Phase adsorbiert bleiben, deutlich besser aus dem System entfernt, wobei ein deutlich höherer Reinigungseffekt für die Nukleinsäuren erzielt wird.

Dem Fachmann ist die chemische Struktur der Nukleinsäuren im Sinne dieser Erfindung bekannt. Es werden unter Nukleinsäuren sowohl native Desoxyribonukleinsäuren (im Weiteren als DNA bezeichnet), wie auch Ribonukleinsäuren (im Weiteren als RNA bezeichnet) mit einer Kettenlänge von mehr als 20 Basenpaaren, insbesondere mehr als 100 Basenpaaren verstanden.

Dabei ist der chemische bzw. biologische Ursprung der Nukleinsäuren nicht für die Möglichkeit ihrer Isolierung nach dem erfindungsgemäßen Verfahren von Belang.
Es können Nukleinsäuren aus biologischen Flüssigkeiten wie beispielsweise Blut, Plasma, Serum oder Bakterienkulturen isoliert werden. Die biologische Rolle der Nukleinsäuren ist dabei unerheblich. Es können beispielsweise sowohl genomische Nukleinsäuren aus Zellkernen, wie auch Nukleinsäuren aus Organellen, etwa Mitochondrien, nach dem erfindungsgemäßen Verfahren isoliert werden.

Manchmal ist es von Vorteil, insbesondere bei der Isolierung von Nukleinsäuren aus biologischen Materialien, die ursprünglichen Probenmaterialien mit Hilfe von enzymatischen Prozessen, mechanischen oder thermischen Behandlungen oder Kombinationen dieser Verfahren, vor der eigentlichen Isolierung aufzuschließen und die Nukleinsäuren leichter der Isolierung zugänglich zu machen.

Ebenso ist neben der Isolierung von Nukleinsäuren aus biologischen Materialien die Isolierung von Nukleinsäuren aus Mischungen und Rezepturen zur in vitro Manipulation von Nukleinsäuren möglich. Beispielhaft seien hier erwähnt Reaktionsgemische zur DNA Sequenzierung, Reaktionsansätze der Polymerasekettenreaktion (PCR), Reaktionsansätze zur Spaltung von Nukleinsäuren mit Restriktionsendonukleasen.

Die Einführung der funktionellen Gruppen auf das im erfindungsgemässen Verfahren verwendete Adsorbens führt dabei zu Oberflächeneigenschaften des Adsorbens, die klar von denen des Ursprungsmaterials zu unterscheiden sind.

Die isolierten Nukleinsäuren sind in der PCR und anderen molekularbiologischen Analysenverfahren einsetzbar.

In einer besonderen Ausführungsform der Erfindung entfällt die Elution der Nukleinsäuren von der modifizierten Oberfläche und die an die Oberfläche gebundenen Nukleinsäuren werden direkt und ohne eluiert zu werden in die molekularbiologische Analytik eingebracht.

Gegenstand der Erfindung ist auch ein Testkit zur Durchführung des Verfahrens.
Er umfasst
- ein Trägermaterial, das eine Oberfläche aufweist, die mindestens zwei chemisch unterscheidbare, nicht zu einer Stoffklasse gehörende funktionelle Gruppen "X" und "Y" besitzt, die beide eine sehr schwache bis keine (inherente) Affinität zu Nukleinsäuren besitzen,
- sowie die zur Isolierung notwendigen chemischen Substanzen, bevorzugt in Form von gebrauchsfertigen Puffern und Suspensionen.

Die Erfindung soll nun anhand von einigen Beispielen erklärt werden, ohne sie jedoch auf diese Beispiele zu beschränken.

### Ausführungsbeispiele

### Beispiel 1:

### Herstellung von aminopropyliertem Kieselgel

10g magnetisches Kieselgel (AGOWA GmbH) werden 24 Stunden in einer 1%igen Lösung von Aminopropyl-triethoxysilan in Ethanol am Rückfluß gekocht.
Dann saugt man das modifizierte Kieselgel ab, wäscht zweimal mit Ethanol, trocknet an der Luft und erhitzt anschließend 4h auf 110°C im Trockenschrank.

### Beispiel 2:

### Einführung von Karboxylgruppen auf aminopropyliertes magnetisches Kieselgel ("X" = Si-OH, "Y" = COOH)

5g des im Beispiel 2 hergestellten Kieselgels werden in 100ml 0.05 M Acetatpuffer mit einem pH von 5 suspendiert und 1 g Bersteinsäureanhydrit zugegeben. Das Gemisch rührt man 2h, saugt den Feststoff ab und wäscht anschließend neutral. Nun wird der Filterkuchen an der Luft getrocknet und steht zur Nukleinsäureisolation zur Verfügung.

Dann saugt man das Kieselgel ab, wäscht es dreimal mit ca. 100ml Wasser und trocknet es an der Luft.

### Beispiel 3

### Herstellung magnetischer Karboxymethylcellulose ("X" = C-OH, "Y" = COOH)

1 g Karboxymethylzellulose werden in 50ml 10% Tetramethylammoniumhydroxid gelöst. Dazu gibt man 200mg Eisen-(II,III)-oxidsol und rührt eine Stunde bei Raumtemperatur.
Nun wird die Mischung mit 2M Natriumacetatpuffer, pH 5 neutralisiert und die ausgefallende magnetische Karboxymethylzellulose abgetrennt, mit Wasser neutral gewaschen und an der Luft getrocknet.

### Beispiel 4

### Herstellung von Adsorptionsmaterial bestehend aus einem Gemisch von Polyacrylsäure und vernetztem Polyvinylalkolhol ("X" = C-OH, "Y" = COOH)

Eine Mischung die 5g Polyvinylalkohol (Molmasse 22,000 g/mol), 20g Tween 20, 1g Polyacrylsäure (Molmasse 250,000 g/mol) und 74g Wasser enthält, wird mit Salzsäure auf pH 1.5 gebracht. Anschließend addiert man 50ml 5%ge Glutardialdehydlösung unter heftigem Rühren und rührt nach Ende der Zugabe noch eine weitere Stunde bei Raumtemperatur nach.
Das gelartig flockige Material wird abgetrennt und mit Wasser neutral gewaschen. Dann trocknet man das Gel und mörsert es auf die gewünschte Korngröße.

### Beispiel 5:

### Isolierung von DNA aus humanem EDTA Blut

10µl Blut werden mit 90µl Lysepuffer (1% SDS, 50mM TrisHCl, 0,1 M NaCl, 20mM EDTA, pH 8) und 5µl Proteinase K Lösung (40µg/ml) gemischt und 10 Minuten bei 55°C inkubiert.
Das Lysat wird zu einer Mischung aus 15µl einer Suspension der im Beispiel 3 hergestellten Partikel (100mg Partikel in 1ml Wasser) und 300 ml Bindungspuffer (2M Guanidinhydrochlorid, 10% Laurylsarcosin-Natriumsalz, 30 vol% Ethanol, 20mM BisTris pH 6,5)) gegeben. Alle Komponenten werden sorgfältig gemischt und 2 Minuten bei Raumtemperatur inkubiert.
Dann separiert man die Magnetpartikel mit der gebundenen DNA und wäscht mit 500µl 3mM Spermidinhydrochloridlösung in 10mM Kaliumphosphatpuffer, pH 6,5 und anschließend mit 500µl destilliertem Wasser.

Die an die Partikel gebundene DNA wird dann in 50µl TE (10mM TrisHCl pH 8, 1 mM EDTA) im Verlauf von 10 Minuten bei 55°C eluiert.

### Beispiel 6:

### Isolation von DNA aus Pflanzenmaterialien

Ca. 30mg pflanzliche Keimblätter werden mit 250µl eines Lysepuffers (1% CTAB, 10mM TrisHCl pH 8, 50mM EDTA, 0,1 M Natriumchlorid) versetzt und in einer Kugelmühle gemahlen. Anschließend inkubiert man das Mahlgut für 10 Minuten bei 55 Grad und zentrifugiert dann die festen Bestandteile ab. 200µl des von Feststoffen freien Lysates werden mit 30µl der Partikelsuspension und 500µl des Bindungspuffers, die im Beispiel 5 verwendet wurden, versetzt und alle Komponenten gut durchmischt.
Nach Abtrennen der an die Magnetpartikel gebundenen DNA wird wiederum mit 500µl Waschpuffer (3mM Spermidinhydrochloridlösung in 10mM Kaliumphosphatpuffer) und anschließend mit 500µl Wasser gewaschen.

Die an die Partikel gebundene DNA wird dann in 100µl TE (10mM TrisHCl pH 8, 1 mM EDTA) im Verlauf von 10 Minuten bei 55 Grad eluiert.

### Beispiel 7:

### DNA Isolation aus einem Mundschleimhautabstrich

Ein Wattetupfer, der zur Isolation von Schleimhautzellen aus dem Mund benutzt wurde, wird in 150µl Lysepuffer (1 % SDS, 50mM TrisHCl, 0,1 M NaCl, 20mM EDTA, pH 8), der 5µl einer Proteinase K Lösung (4mg/ml Wasser) enthält, gesteckt und für 10 Minuten bei 55°C inkubiert. Das Inkubationsgefäß wird mit einem kleinen Loch am Boden versehen und in ein Auffanggefäß gesteckt. Durch Zentrifugation trennt man die flüssigen Bestandteile des Lysates ab. 100µl des Lysates werden zu einer Mischung aus 15µl einer Suspension der im Beispiel 4 hergestellten Partikel (100mg Partikel in 1ml Wasser) und 300 ml Bindungspuffer (2M Guanidinhydrochlorid, 10% Laurylsarcosin-Natriumsalz, 30 vol% Ethanol, 20mM BisTris pH 6,5) gegeben. Alle Komponenten werden sorgfältig gemischt und 2 Minuten bei Raumtemperatur inkubiert.
Dann separiert man die Magnetpartikel mit der gebundenen DNA und wäscht mit 500µl 3mM Spermidinhydrochloridlösung in 10mM Kaliumphosphatpuffer, pH 6,5 und anschließend mit 500µl destilliertem Wasser.

Die an die Partikel gebundene DNA wird dann in 50µl TE (10mM TrisHCl pH 8, 1 mM EDTA) im Verlauf von 10 Minuten bei 55 Grad eluiert.

### Beispiel 8:

### DNA Isolation aus einem Mundschleimhautabstrich

Ein Wattetupfer, der zur Isolation von Schleimhautzellen aus dem Mund benutzt wurde, wird in 150µl Lysepuffer (1 % SDS, 50mM TrisHCl, 0,1 M NaCl, 20mM EDTA, pH 8), der 5µl einer Proteinase K Lösung (4mg/ml Wasser) enthält, gesteckt und für 10 Minuten bei 55°C inkubiert. Das Inkubationsgefäß wird mit einem kleinen Loch am Boden versehen und in ein Auffanggefäß gesteckt. Durch Zentrifugation trennt man die flüssigen Bestandteile des Lysates ab. 100µl des Lysates werden zu einer Mischung aus 15µl einer Suspension der im Beispiel 4 hergestellten Partikel (100mg Partikel in 1ml Wasser) und 200 ml iso-Propanol gegeben. Alle Komponenten werden sorgfältig gemischt und 2 Minuten bei Raumtemperatur inkubiert.
Dann separiert man die Magnetpartikel mit der gebundenen DNA und wäscht mit 500µl 1 mM Spermidinhydrochloridlösung in 10mM Kaliumphosphatpuffer, pH 6,5 und anschließend mit 500µl destilliertem Wasser.

Die an die Partikel gebundene DNA wird dann in 50µl TE (10mM TrisHCl pH 8, 1mM EDTA) im Verlauf von 10 Minuten bei 55 Grad eluiert.

### Beispiel 9:

### Isolation von DNA aus Pflanzenmaterialien

Ca. 30mg Blätter von einer Tomatenpflanze werden mit 250µl eines Lysepuffers (1 % CTAB, 10mM TrisHCl pH 8, 50mM EDTA, 0,1 M Natriumchlorid) versetzt und in einer Kugelmühle gemahlen. Anschließend inkubiert man das Mahlgut für 10 Minuten bei 65 Grad und zentrifugiert dann die festen Bestandteile ab. 200µl des von Feststoffen freien Lysates werden mit 30µl der Partikelsuspension und 400µl Ethanol versetzt und gut durchmischt. Nach Abtrennen der an die Magnetpartikel gebundenen DNA wird wiederum mit 500µl Waschpuffer (3mM Spermidinhydrochloridlösung in 10mM Kaliumphosphatpuffer) und anschließend mit 500µl Wasser gewaschen.

Die an die Partikel gebundene DNA wird dann in 100µl TE (10mM TrisHCl pH 8, 1 mM EDTA) im Verlauf von 10 Minuten bei 55 Grad eluiert.

## Patentansprüche

1. Verfahren zur Isolation von Nukleinsäuren, **dadurch gekennzeichnet, dass** zur Bindung der Nukleinsäuren ein Trägermaterial verwendet wird, das eine Oberfläche aufweist, die mindestens zwei chemisch unterscheidbare, nicht zu einer Stoffklasse gehörende funktionelle Gruppen "X" und "Y" besitzt, die beide eine sehr schwache bis keine (inherente) Affinität zu Nukleinsäuren besitzen, wobei ein die Nukleinsäuren enthaltendes Probenmaterial mit dem Trägermaterial inkubiert wird und die Bedingungen so gewählt werden, dass beide funktionellen Gruppen unterschiedliche Affinitäten zu den Nukleinsäuren haben und die Nukleinsäure nacheinander an die funktionelle Gruppe "X" und "Y" gebunden wird.

2. Verfahren nach Anspruch 1, **dadurch gekennzeichnet, dass** die auf dem Trägermaterial vorhandenen funktionellen Gruppen "X" zu polaren Wechselwirkungen, vorzugsweise im Sinne von van der Waals Wechselwirkungen, und/oder zur Ausbildung von Wasserstoffbrückenbindungen fähig sind, vorzugsweise stellt die Gruppe "X" gebundene Hydroxyl- oder Mercaptogruppen oder Derivate dieser Gruppen dar.

3. Verfahren nach Anspruch 1 oder 2, **dadurch gekennzeichnet, dass** die auf den Trägermaterialien vorhandenen funktionellen Gruppen "Y" zur Ausbildung von ionischen Wechselwirkungen fähig sind, vorzugsweise sind es schwache ionenaustauschaktive organische Gruppen ausgewählt aus Karbonsäuregruppen, Borsäuregruppen oder Phosphorsäuregruppen.

4. Verfahren nach einem der Ansprüche 1 bis 3, **dadurch gekennzeichnet, dass** die Bindung der zu isolierenden Nukleinsäuren an die Gruppe "X" unter Ausbildung polarer Wechselwirkungen erfolgt, wobei die Polarität der die zu isolierende Nukleinsäure enthaltenden Probe niedriger gewählt wird als die des Trägermaterials.

5. Verfahren nach Anspruch 4, **dadurch gekennzeichnet, dass** die Einstellung der Polarität zur Adsorption der Nukleinsäuren an die Gruppen "X" durch die Einwirkung von anorganischen oder organischen Salzen in Kombination mit ionischen oder nichtionischen Detergenzien und/oder der Zugabe von mit Wasser mischbaren organischen Lösungsmitteln, vorzugsweise mit Wasser mischbaren Alkoholen, erfolgt.

6. Verfahren nach einem der Ansprüche 1 bis 5, **dadurch gekennzeichnet, dass** die Bindung der zu isolierenden Nukleinsäuren an die Gruppe "Y" durch Änderung der chemischen Umgebung erfolgt, wobei die Spezifität der gebundenen Nukleinsäure zur funktionellen Gruppe "X" des Trägermaterials aufgehoben und eine Spezifität zur Gruppe "Y" aufgebaut wird, vorzugsweise unter Ausbildung ionischer Wechselwirkungen.

7. Verfahren nach einem der Ansprüche 1 bis 6, **dadurch gekennzeichnet, dass** die Änderung der chemischen Umgebung durch die Einwirkung von bifunktionellen Reagenzien, die sowohl eine Affinität zur Gruppe "Y" als auch zu den an "X" gebundenen Nukleinsäuren hat, erfolgt.

8. Verfahren nach einem der Ansprüche 1 bis 7, **dadurch gekennzeichnet, dass** als bifunktionelle Reagenzien oligomere Amine, vorzugsweise Derivate oligomerer Ethylenamine mit 2 bis 6 Aminogruppen, eingesetzt werden.

9. Verfahren nach einem der Ansprüche 1 bis 8, **dadurch gekennzeichnet, dass** die bifunktionellen Reagenzien ausgewählt sind aus Spermin, Spermidin oder Putrescin und deren Derivate, vorzugsweise ihre Hydrochloride.

10. Verfahren nach einem der Ansprüche 1 bis 9, **dadurch gekennzeichnet, dass** die bifunktionellen Reagenzien in einer Konzentration von 0,1 bis 20 mM, vorzugsweise in einer Konzentration von 0,5 bis 3 mM eingesetzt werden.

11. Verfahren nach einem der Ansprüchen 1 bis 10, **dadurch gekennzeichnet, dass** als funktionalisierte Trägematerialien Mikropartikeln mit superparamagnetischen Eigenschaften mit einer Größe von 0,1 bis 100µm eingesetzt werden.

12. Verfahren nach einem der Ansprüche 1 bis 11, **dadurch gekennzeichnet, dass** als funktionalisierte Trägermaterialien flächenmäßig strukturierte Oberflächen, vorzugsweise Mikroreaktionsgefäße oder strukturierte Mikrofluidiksysteme mit vorzugsweise funktionsbezogenen Mikrokanälen, deren Oberfläche über geeignete chemische oder physikalische Verfahren mit den funktionellen Gruppen "X" und "Y" modifiziert wurden, eingesetzt werden.

13. Verfahren nach einem der Ansprüche 1 bis 12, **dadurch gekennzeichnet, dass** die zu isolierenden Nukleinsäuren, vorzugsweise in Lösungen, die eine Salzkonzentration von kleiner 100mM organischer oder anorganischer Salze und einen pH-Wert von größer 7,5 haben, keine oder nur eine sehr schwache Affinität zu den Trägermaterialien haben, eluiert werden.

14. Verfahren nach einem der Ansprüche 1 bis 13, **dadurch gekennzeichnet, dass** zur Bindung der Nukleinsäuren ein Trägermaterial verwendet wird, das eine Oberfläche aufweist, die als funktionelle Gruppen "X" gebundene Hydroxyl- oder Mercaptogruppen oder Derivate dieser Gruppen aufweist und als funktionelle Gruppen "Y" schwache ionenaustauschaktive organische Gruppen ausgewählt aus Karbonsäuregruppen, Borsäuregruppen oder Phosphorsäuregruppen, wobei ein die Nukleinsäuren enthaltendes Probenmaterial, ggf. nach einem chemischen, enzymatischen und/oder mechanischen Aufschluss, mit dem Trägermaterial inkubiert wird und
a) die zu isolierende Nukleinsäure unter Zugabe von anorganischen oder organischen Salzen in Kombination mit ionischen oder nichtionischen Detergenzien und/oder der Zugabe von mit Wasser mischbaren organischen Lösungsmitteln unter Einstellung einer Polarität, die niedriger als die des Trägermaterials ist, an die funktionelle Gruppe "X" gebunden wird,
b) die Nukleinsäure von der Gruppe "X" gelöst und an die Gruppe "Y" unter Änderung des pH-Wertes und/oder der Temperatur, ggf. in Gegenwart eines oligomeren Polyamins gebunden wird, und
c) für die Isolierung notwendige Vorbereitungs- und/oder Waschschritte gleichzeitig oder nacheinander an der an dem Trägermaterial gebundenen Nukleinsäure durchgeführt werden.

15. Testkit zur Durchführung eines Verfahrens gemäß einem der Ansprüchen 1 bis 14 umfassend
- ein Trägermaterial, das eine Oberfläche aufweist, die mindestens zwei chemisch unterscheidbare, nicht zu einer Stoffklasse gehörende funktionelle Gruppen "X" und "Y" besitzt, die beide eine sehr schwache bis keine Affinität zu Nukleinsäuren besitzen,
- sowie die zur Isolierung notwendigen chemischen Substanzen, bevorzugt in Form von gebrauchsfertigen Puffern und Suspensionen.
